(19) Europäisches Patentamt
European Patent Office
Office européen des brevets

(11) EP 1 427 840 B1

(12) **FASCICULE DE BREVET EUROPEEN**

(45) Date de publication et mention de la délivrance du brevet:
**04.10.2006 Bulletin 2006/40**

(21) Numéro de dépôt: **02774892.0**

(22) Date de dépôt: **30.08.2002**

(51) Int Cl.:
*C12P 13/04* (2006.01) *C12P 41/00* (2006.01)
*C12N 9/80* (2006.01)

(86) Numéro de dépôt international:
**PCT/FR2002/002976**

(87) Numéro de publication internationale:
**WO 2003/020943 (13.03.2003 Gazette 2003/11)**

(54) **PROCEDE ENZYMATIQUE POUR LA RESOLUTION ENANTIOMERIQUE D'ACIDES AMINES**

ENZYMATISCHES VERFAHREN ZUR ENANTIOMEREN TRENNUNG VON AMINOSÄUREN

ENZYMATIC METHOD FOR THE ENANTIOMERIC RESOLUTION OF AMINO ACIDS

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **04.09.2001 FR 0111431**
**20.11.2001 US 331613 P**

(43) Date de publication de la demande:
**16.06.2004 Bulletin 2004/25**

(73) Titulaire: **Aventis Pharma S.A.**
**92160 Antony (FR)**

(72) Inventeurs:
- **SALAGNAD, Christophe**
  **F-94490 Ormesson sur Marne (FR)**
- **GOBERT, Claude**
  **F-94240 L'Hay les Roses (FR)**
- **DURY, Marie-Odile**
  **F-94480 Ablon (FR)**

(74) Mandataire: **Bouvet, Philippe**
**Aventis Pharma S.A.**
**Direction des Brevets,**
**Tri LEO/144**
**20, avenue Raymond Aron**
**92165 Antony Cedex (FR)**

(56) Documents cités:
**WO-A-98/50575**

- **SHIBUYA Y ET AL: "ISOLATION AND PROPERTIES OF 7-BETA-(4 CARBOXYBUTANAMIDO) CEPHALOSPORANIC-ACID ACYLASE-PRODUCING BACTERIA" AGRICULTURAL AND BIOLOGICAL CHEMISTRY, vol. 45, no. 7, 1981, pages 1561-1567, XP002198650 ISSN: 0002-1369**
- **SOLOSHONOK V A ET AL: "Biocatalytic Approach to Enantiomerically Pure beta-Amino Acids" TETRAHEDRON: ASYMMETRY, ELSEVIER SCIENCE PUBLISHERS, AMSTERDAM, NL, vol. 6, no. 7, 1 juillet 1995 (1995-07-01), pages 1601-1610, XP004048135 ISSN: 0957-4166 cité dans la demande**
- **RAIMONDI S ET AL: "Kinetic resolutions of racemic amines and alcohols catalyzed by an industrial glutaryl-7-aminocephalosporanic acid acylase with unexpected broad substrate specificity" TETRAHEDRON ASYMMETRY, vol. 14, no. 9, 2 mai 2003 (2003-05-02), pages 1091-1094, XP004420949 ISSN: 0957-4166**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 427 840 B1

## Description

La présente invention se rapporte à un nouveau procédé enzymatique permettant la résolution énantiomérique d'acides aminés sous forme de mélange racémique.

Les acides aminés sont fréquemment utilisés dans toutes sortes d'industries soit par exemple en tant que composés biologiquement actifs, soit en tant qu'intermédiaires de synthèse pour la préparation de composés à visée notamment pharmaceutique, chimique ou agricole. Il est ainsi rapidement apparu qu'il était souvent nécessaire de pouvoir disposer de l'un ou l'autre énantiomère optiquement actif de ces acides aminés. De nombreuses voies de séparation des énantiomères d'acides aminés pro-chiraux ont donc été mise au point. En particulier, des procédés enzymatiques permettant leur résolution énantiomérique se sont révélés être une alternative intéressante aux approches synthétiques asymétriques.

Ainsi, Soloshonok et al. (*Tetrahedron* : *Assymetry,* Vol. 6(7), 1995, pp. 1601-1610) ont mis en oeuvre un procédé enzymatique de résolution énantiomérique d'acides aminés β selon le schéma réactionnel suivant :

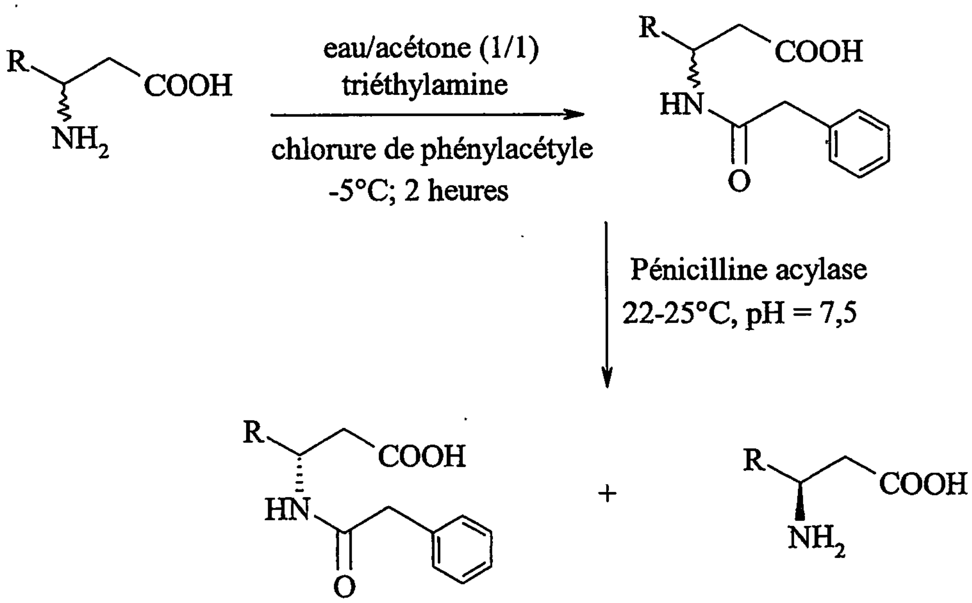


De façon analogue, Topgi et al. (*Bioorg. & Med. Chem.* 1999, Vol. 7, pp. 2221-2229) ont élaboré un procédé de résolution des énantiomères (R) et (S) du 3-amino 4-pentynoate d'éthyle sous forme enantiomériquement pure selon l'un des schémas réactionnels suivants :

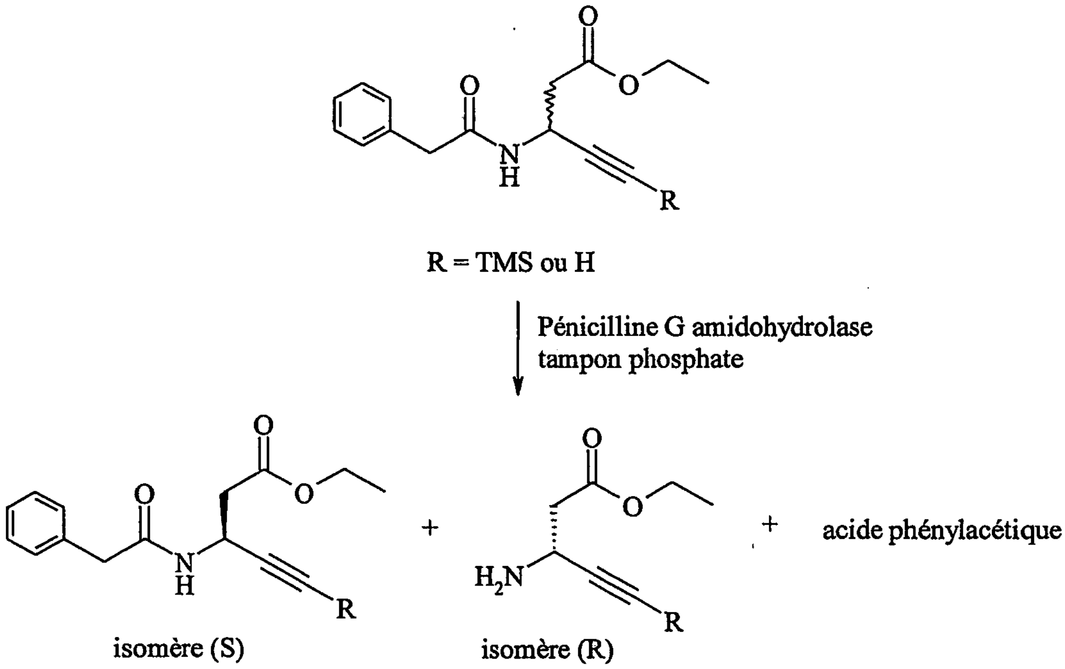


le phénylacétamide de départ étant obtenu par acylation de l'amine correspondante par action de l'acide phénylacétique, ou bien :

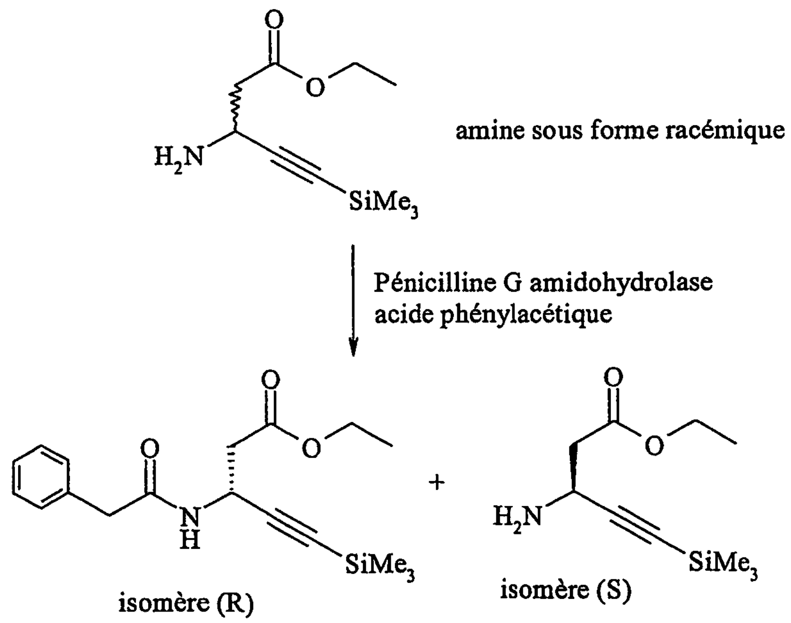


La demande de brevet WO 98/50575 décrit de façon plus générale une méthode de préparation d'un acide aminé β chiral qui comprend la mise en contact d'un mélange racémique dudit acide aminé avec un donneur d'acyle et l'enzyme Pénicilline G acylase (ou amidohydrolase), dans des conditions appropriées pour acyler stéréosélectivement l'un des énantiomères du mélange racémique de l'acide aminé β en son dérivé N-acylé correspondant, l'énantiomère opposé de l'acide aminé β étant obtenu sous une forme enrichie énantiomériquement, selon la schéma réactionnel suivant :

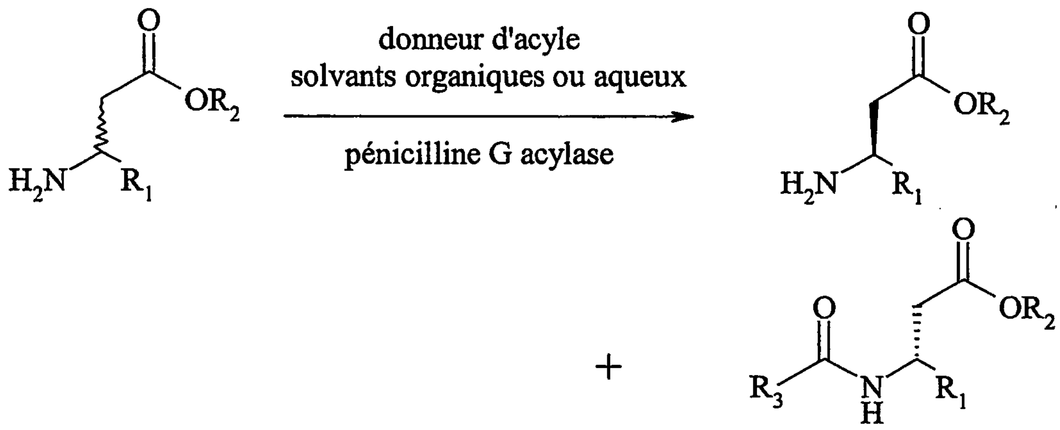


le « donneur d'acyle » en question étant de formule générale :

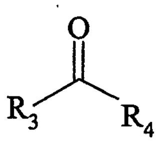

dans laquelle $R_3$ est choisi parmi le phényle, phénoxy, amino, divers dérivés du phényle et la pyridyle, et $R_4$ est choisi parmi les substituants hydroxy, alkoxy, alkyle, alcényle, alcynyle, halogénoalkyle, anyle, anylalkyle, les sucres ou les stéroïdes.

La demande de brevet WO 98/50575 décrit également une autre alternative pour la préparation d'un acide aminé β chiral qui comprend la mise en contact d'un amide sous forme racémique avec l'enzyme Pénicilline G acylase dans des conditions appropriées pour déacyler sélectivement l'un des énantiomères de l'amide sous forme racémique en son acide aminé β correspondant, l'énantiomère opposé de l'amide étant obtenu sous une forme enrichie énantiomériquement, selon la schéma réactionnel suivant :

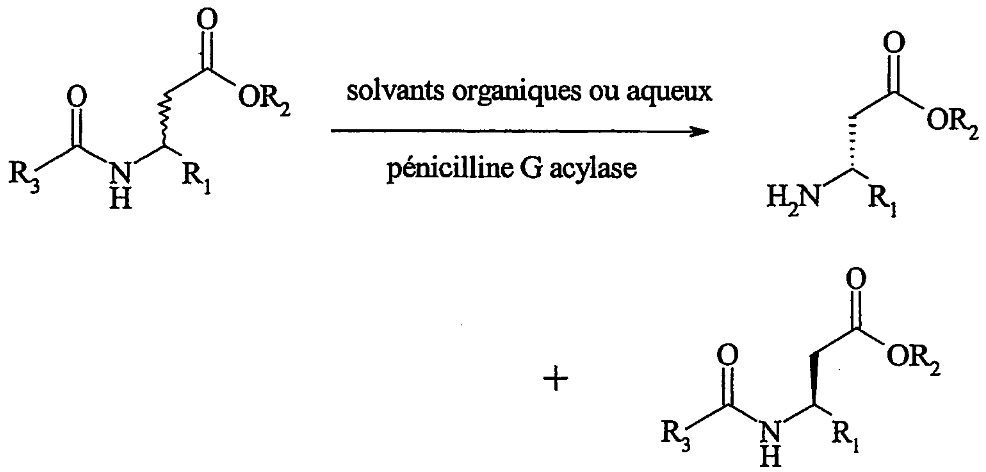


Les procédés discutés ci-avant présentent toutefois l'inconvénient majeur de passer, préalablement ou simultanément à l'étape enzymatique en tant que telle, par un amide intermédiaire dont la formule pourrait être résumée de la façon suivante :

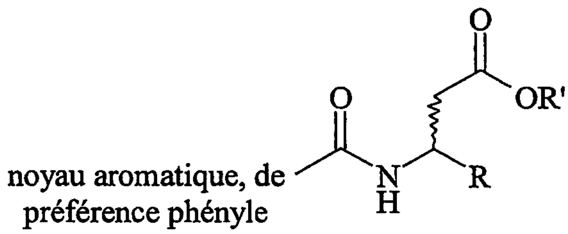

Un tel amide est insoluble en milieu aqueux du fait de la présente du noyau aromatique, ce qui présente des inconvénients. Par exemple, il est connu que certaines enzymes sont solubles en milieu aqueux et sont souvent sensibles à la présence de solvants organiques. Or, pour que le rendement de la réaction enzymatique puisse être optimisé, il est important d'avoir une bonne solubilité du substrat par rapport à l'enzyme de façon à ce qu'ils puissent être en contact intime.

C'est principalement cet inconvénient que la présente invention se propose de résoudre. En effet, la présente invention concerne selon un premier aspect un nouveau procédé de séparation des énantiomères d'un acide aminé qui consiste à traiter un mélange racémique dudit acide aminé avec de l'anhydride glutarique puis avec l'enzyme glutaryl 7-ACA acylase de sorte que l'on récupère l'un des énantiomères dudit acide aminé, l'autre énantiomère restant sous forme de dérivé de l'amide glutaryle correspondant.

Ce procédé est particulièrement avantageux car l'emploi d'anhydride glutarique permet de passer intermédiairement par un dérivé de l'amide glutaryle correspondant à l'acide aminé de départ, la fonction glutaryle conférant à la molécule sa solubilité en milieu aqueux. Il en résulte que le procédé selon la présente invention peut être mis en oeuvre dans des conditions réactionnelles douces et en milieu aqueux, c'est-à-dire en particulier sans l'usage d'un co-solvant organique.

Le procédé selon la présente invention possède également l'avantage de s'appliquer globalement à toutes les formes d'acides aminés ($\alpha$, $\beta$, $\gamma$...). Au sens de la présente invention, on regroupe sous le terme générique « acide aminé » aussi bien les acides aminés en tant que tel (à savoir les composés ayant une fonction amino et une fonction acide -COOH) que les dérivés esters correspondant (à savoir les composés pour lesquels la fonction acide est remplacée par une fonction ester COOR). De façon préférée, le procédé selon l'invention s'applique plus précisément aux acides aminés de formule générale (I) :

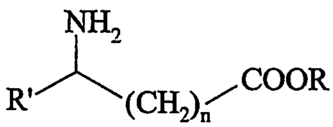

(I)

dans laquelle

- n est un entier choisi parmi 0, 1, 2, 3, 4, 5 ou 6,
- R représente un atome d'hydrogène ou bien un radical alkyle, alcène, alcyne, cycloalkyle, aryle, un hydrocarbure polycyclique condensé, ou un hétérocycle, tous ces radicaux étant éventuellement substitués,
- et R' représente un radical alkyle, alcène, alcyne, cycloalkyle, aryle, un hydrocarbure polycyclique condensé, un hétérocycle, ou bien un radical oxy, thio, sulfoxyde ou sulfonyle substitué par un groupe alkyle, aryle, cycloalkyle ou un hétérocyle, tous ces radicaux étant en outre éventuellement substitués.

Ainsi, dans le cas où les acides aminés sont de formule générale (I), le procédé selon la présente invention peut être représenté par le schéma réactionnel de la figure 1. Ce schéma représente une première étape de traitement par l'anhydride glutarique et une seconde étape de traitement par l'enzyme glutaryl 7-ACA acylase. La configuration de chacun des produits obtenus, à savoir l'acide aminé d'une part et le dérivé de l'amide glutaryle d'autre part, dépend de la nature du radical R'.

Au sens de la présente invention, les radicaux alkyles, alcènes et alcynes contiennent en général entre 1 et 30 atomes de carbone en chaîne droite ou ramifiée, sans que cela ne soit pour autant limitatif. Cela s'applique également aux cas où ces radicaux sont des substituants d'autres radicaux. De préférence, ces radicaux contiennent entre 1 et 20 atomes de carbone en chaîne droite ou ramifiée, et encore plus préférentiellement, entre 1 et 10 atomes de carbone

en chaîne droite ou ramifiée. Les radicaux alkyles peuvent par exemple être choisis parmi : méthyle, éthyle, n-propyle, n-butyle, n-pentyle, n-hexyle, n-heptyle, n-octyle, n-nonyle, n-décyle, n-undécyle, n-dodécyle, n-tridécyle, n-pentadécyle, n-hexadécyle, n-heptadécyle, n-octadécyle, isopropyle, isobutyle, isopentyle, isohexyle, 3-méthylpentyle, néopentyle, néohexyle, 2,3,5-triméthylhexyle, sec-butyle, tert-butyle, tert-pentyle. Des radicaux alkyls préférés sont par exemple méthyle, éthyle, n-propyle, isopropyle, n-butyle, isobutyle, sec-butyle, tert-butyle, n-pentyle, isopentyle, n-hexyle et isohexyle. Les radicaux alcènes peuvent par exemple être choisis parmi le vinyle, le 1-propènyle, l'allyle, le butènyle, le 2-méthyl-1-propènyle, le 2-méthyl-2-propènyle ou le 3-méthyl-2-butènyle. Les radicaux alcynes peuvent par exemple être choisis parmi l'éthynyle, le 1-propynyle ou le propargyle.

Au sens de la présente invention, les radicaux cycloalkyles contiennent en général entre 3 et 12 atomes de carbone. De préférence, il sont choisis parmi le cyclopropyle, le cyclobutyle, le cyclopentyle, le cyclohexyle, le cycloheptyle, le cyclooctyle, le cyclononyle, le cyclodécyle, le cycloundécyle et le cyclododécyle. Selon un autre aspect de la présente invention, les cycloalkyles peuvent être polycycliques. De préférence, ces radicaux sont choisis parmi les bicycloalkyles ou les tricycloalkyles.

Selon la présente invention, on entend par radical « aryle » les radicaux hydrocarbonés aromatiques univalents. Parmi les aryles, le phényle éventuellement substitué est préféré.

Au sens de la présente invention, on entend par hydrocarbure polycyclique condensé, les radicaux choisis de préférence parmi le pentalène, l'indène, le naphtalène, l'azulène, l'heptalène, le biphénylène, l'*as*-indacène, le *s*-indacène, l'acénaphtylène, le fluorène, le phénalène, le phénanthrène, l'anthracène, le fluoranthène, l'acéphénanthrylène, l'acéanthrylène, letriphénylène, le pyrène, le chrysène, le naphtacène, le pléiadène, le picène, le pérylène, le pentaphène, le pentacène, le tétraphénylène, l'hexaphène, l'hexacène, le rubicène, le coronène, le trinaphtylène, l'heptaphène, l'heptacène, le pyranthrène ou l'ovalène.

Au sens de la présente invention, le terme « hétérocycle » désigne les composés monocycliques ou polycycliques fusionnés et contenant un ou plusieurs hétéroatomes, chaque cycle étant formé de 3 à 10 chaînons. De préférence, les hétérocycles selon la présente invention contiennent entre 1 et 3 hétéroatomes choisis parmi l'oxygène, le soufre et l'azote dans un cycle formé de 3 à 10 chaînons. Les hétérocycles de la présente invention sont choisis de préférence parmi le thiophène, le benzo[b]thiophène, le naphto[2,3-b]thiophène, le thianthrène, le furanne, le 2 H-pyranne, l'isobenzofuranne, le 2 *H*-chromène, le xanthène, la phénoxathiine, le 2 *H*-pyrrole, le pyrrole, l'imidazole, le pyrazole, la pyridine, la pyrazine, la pyrimidine, la pyridazine, l'indolizine, l'iso-indole, le 3 *H*-indole, l'indole, le 1 *H*-indazole, la purine, la 4 *H*-quinolizine, l'isoquinoléine, la quinoléine, la phtalazine, la naphtyridine-1,8, la quinoxaline, la quinazoline, la cinnoline, la ptéridine, la 4a *H*-carbazole, le carbazole, la β-carboline, la phénanthridine, l'acridine, la périmidine, la phénanthroline-1,7, la phénazine, la phénarsazine, l'isothiazole, la phénothiazine, l'isoxazole, le furazanne, la phénoxazine, l'isochromane, la pyrrolidine, la Δ2-pyrroline, l'imidazolidine, la Δ2-imidazoline, la pyrazolidine, la Δ3-pyrazoline, la pipéridine, la pipérazine, l'indoline, l'iso-indoline, la quinuclidine et la morpholine.

Au sens de la présente invention, lorsque les différents radicaux tels que définis ci-avant sont substituées, ledit ou lesdits substituants sont choisis en général parmi les atomes d'halogène, les groupes aryle, hétérocycle, hydroxy, alkoxy, aryloxy, thiol, alkylthio, arylthio, alkylsulfoxyde, arylsulfoxyde, alkylsulfonyle, arylsulfonyle, cyano, nitro, sulfonamide, alkylsulfonamide et arylsulfonamide, selon la nature du radical. De préférence, les différents radicaux tels que définis ci-avant sont substituées 1, 2 ou 3 fois. Selon un aspect préféré, les atomes d'halogène sont choisis parmi le chlore, le fluor, le brome et l'iode. Dans le cas où les radicaux alkyles sont substitués par des atomes d'halogène, il s'agit préférentiellement d'atomes de fluor. De préférence, le nombre de substituants fluor est de 1, 2, 3, 4, 5, 6 ou 7. Par exemple, il peut s'agir d'un groupe trifluorométhyle.

De préférence, les acides aminés selon la présente invention sont choisis parmi les composés de formule générale (I) dans laquelle n est un entier choisi parmi 0, 1, 2 ou 3, R représente un atome d'hydrogène ou bien un radical alkyle ou aryle et R' est tel que défini ci-avant.

Encore plus préférentiellement, les acides aminés selon la présente invention sont choisis parmi les composés de formule générale (I) dans laquelle n est un entier égal à 0, 1 ou 2, R représente un atome d'hydrogène ou bien une fonction alkyle et R' est choisi parmi les aryles ou les hétérocycles éventuellement substitués. Dans ce dernier cas, les radicaux aryles et/ou hétérocycles sont de préférence substitués 1, 2 ou 3 fois.

L'enzyme glutaryl 7-ACA acylase a déjà été utilisée comme catalyseur dans de nombreux procédés industriels, notamment pour l'hydrolyse de β-lactames tels que l'acide N-glutaryl 7-aminoacétoxycéphalosporinique. Elle peut être dérivée de nombreux microorganismes, par exemple du genre *Acinetobacter, Arthrobacter, Bacillus, Pseudomonas, Stenotrophomonas* ou encore *Xanthomonas,* selon les techniques bien connues de l'homme de l'art, spécialiste des enzymes. L'enzyme Glutaryl 7-ACA acylase peut également être obtenue commercialement par exemple auprès de Roche Diagnostic GmbH (Roche Molecular Biochemicals, Standhofer Strasse 116, D-68305 Mannheim) ou encore auprès de Recordati S.p.A. (Stabilimento di opera, Via Lambro 38, I-20090 Opera (MI)).

L'enzyme glutaryl 7-ACA acylase peut être utilisée sous différentes formes sans que cela ne modifie sa stéréospécificité et sa stéréosélectivité. Par exemple, la glutaryl 7-ACA acylase peut être utilisée sous forme soluble ou bien immobilisée. Dans ce second cas, l'enzyme est généralement immobilisée selon des techniques bien connues de

l'homme de l'art. Par exemple, l'enzyme peut être contenue dans des gels polymériques ou bien attachée à des supports solides par liaison covalente, réticulation, adsorption ou encapsulation. Des supports adaptés et qui sont couramment utilisés sont par exemple le verre poreux, les céramiques poreuses, les polymères synthétiques (par exemple le polystyrène, les polyvinylalcools, le polyéthylène, les polyamides ou les polyacrylamides), ou les polymers d'origine naturelle (par exemple la cellulose).

Par l'emploi de l'enzyme glutaryl 7-ACA acylase, il est possible d'obtenir un excès énantiomérique (« ee ») élevé pour chaque énantiomère, en particulier supérieur ou égal à 90 %, voire même supérieur ou égal à 95 %, et de préférence supérieur ou égal à 99 %. Au sens de la présente invention, on entend par « excès énantiomérique » l'excès en % de l'un des énantiomères par rapport au mélange racémique. Plus précisément, l'excès énantiomérique se calcule de la façon suivante :

$$ee\,(\%) = \frac{[R] - [S]}{[R] + [S]} * 100 = \%\,(R) - \%\,(S)$$

[R] et [S] représentant la concentration en énantiomère (R) et (S) respectivement.

Généralement, la quantité d'enzymes mise en oeuvre par rapport à la quantité totale d'acide aminé de départ (substrat) est comprise 1 et 100 unités (U) par mmole de substrat, et de préférence entre 10 et 40 unités par mmole de substrat. 1 unité d'enzyme correspond à la quantité d'enzyme nécessaire pour hydrolyser 1 $\mu$mole d'acide N-glutaryl 7-aminoacétoxycéphalosporinique par minute dans des condition standards de pH et température connues de l'homme de l'art.

Selon l'invention, on effectue la réaction en milieu aqueux éventuellement tamponné. Dans ce cas, le tampon aqueux d'une concentration comprise entre 10 mM et 200 mM peut être choisi parmi les tampons acétate utilisables à pH compris entre 5 et 6,5 ou les tampons phosphate utilisables à pH compris entre 6,5 et 8, ou encore les tampons pyrophosphate utilisables à pH compris entre 8 et 9.

Le procédé selon l'invention est ainsi mis en oeuvre dans un milieu où le pH est contrôlé et ajusté entre 6 et 9. De préférence, le pH du milieu réactionnel est contrôlé et ajusté précisément entre 7,5 et 8,5 et encore plus préférentiellement entre 8 et 8,5. Le pH peut être contrôlé à l'aide d'un pH-stat par ajout d'un acide tel que par exemple de l'acide chlorhydrique, de l'acide sulfurique ou de l'acide phosphorique, et d'une base telle que par exemple l'hydroxyde de sodium, l'hydroxyde de potassium ou l'ammoniaque.

Le traitement des acides aminés selon l'invention par l'anhydride glutarique est mis en oeuvre à une température comprise entre 20°C et 40°C, et de préférence entre 25°C et 35°C. En outre, la seconde étape mettant en oeuvre l'enzyme glutaryl 7-ACA acylase est effectuée à une température comprise entre 10°C et 50°C, et de préférence entre 25°C et 35°C.

Enfin, le temps de réaction varie globalement entre 1 heure et 100 heures et dépend notamment de l'acide aminé concerné et de la concentration en enzymes. Généralement, on laisse la réaction se dérouler autant de temps que nécessaire pour obtenir l'énantiomère désiré avec un excès énantiomérique satisfaisant. On contrôle la quantité d'acide aminé chiral obtenue et la valeur de l'excès énantiomérique en utilisant les techniques classiques connues de l'homme du métier. Avantageusement, le contrôle est effectué par CLHP (Chromatographie Liquide Haute Performance).

Selon la présente invention, les énantiomères (R) et (S) obtenu par le procédé décrit ci-avant peuvent être aisément séparées du fait qu'ils se présentent l'un sous forme d'amine soluble en milieu aqueux et l'autre sous forme d'amide solide. Ainsi, un autre objet de la présente invention concerne le procédé tel qu'exposé ci-avant qui comprend comme étape subséquente la séparation des énantiomères (R) et (S).

La séparation desdits énantiomères (R) et (S) peut être aisément effectuée grâce aux techniques classiques connues de l'homme du métier. On opère par exemple par filtration, extraction, chromatographie ou cristallisation.

Dans le cas où l'on souhaite isoler l'autre énantiomère sous forme d'acide aminé et non pas sous forme de dérivé de l'amide glutaryle, ledit énantiomère du dérivé de l'amide glutaryle qui a été isolé selon le procédé décrit ci-avant est ensuite hydrolysé de sorte que l'on récupère l'acide aminé correspondant sous forme d'énantiomère. Il est à noter que ce procédé est avantageux car l'hydrolyse permet de conserver la stéréochimie du composé mis en oeuvre et ne conduit pas à une racémisation du dérivé de l'amide glutaryle chiral. Dans le cas où les acides aminés sont de formule générale (I), cette étape supplémentaire peut être représentée par le schéma réactionnel selon la figure 2.

L'hydrolyse est effectuée conformément aux techniques classiques connues de l'homme du métier. Il peut notamment s'agir d'une hydrolyse acide ou basique. Dans ce dernier cas, on opère par exemple en présence d'une base telle que la soude, à température comprise entre 50°C et 90°C et sous pression atmosphérique. Cependant, toutes autres conditions opératoires également convenables et connues de l'homme de l'art peuvent être utilisées.

La présente invention est utile en ce sens qu'elle permet de résoudre des acides aminés sous forme racémique,

ce qui permet de disposer de l'un ou l'autre énantiomère dudit acide aminé, lesdits énantiomères constituant notamment des intermédiaires de synthèse.

A titre illustratif, la présente invention permet par exemple de disposer de l'acide (S) 3-amino 3-phényl propanoïque qui constitue un intermédiaire dans la synthèse du composé de formule générale :

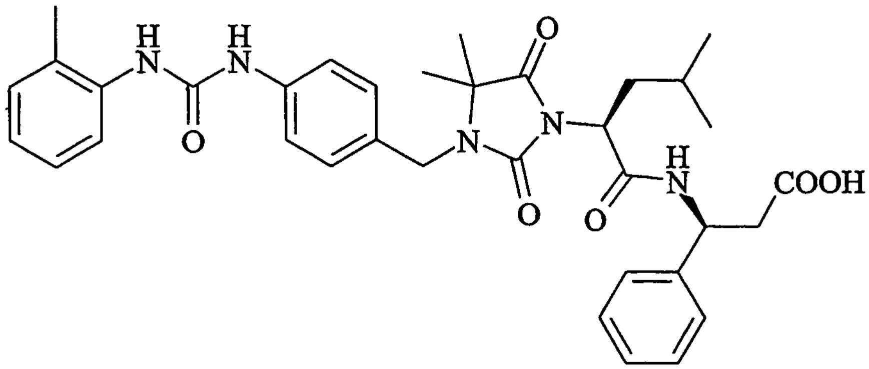

qui est un antagoniste du récepteur VLA4 impliqué notamment dans les maladies de l'asthme.

Outre les dispositions qui précèdent, la présente invention comprend également des caractéristiques et avantages qui ressortiront des exemples qui suivent, et qui doivent être considérés comme illustrant l'invention sans en limiter la portée.

**FIGURES**

**Figure 1 :** Représentation schématique de la 1ère étape de traitement d'un acide aminé de formule générale (I) selon la présente invention par l'anhydride glutarique et de la 2nde étape de traitement par l'enzyme glutaryl 7-ACA acylase. La configuration de chacun des produits obtenus, à savoir l'acide aminé d'une part et le dérivé de l'amide glutaryle d'autre part, dépend de la nature du radical R'.

**Figure 2 :** Représentation schématique de l'étape de transformation du dérivé de l'amide glutaryle chiral en son acide aminé chiral correspondant par hydrolyse.

**EXEMPLES**

**Exemple 1 : Résolution de l'acide 3-amino-3-(4'-nitrophényle) propanoïque sous forme de mélange racémique**

a) Acylation de l'acide 3-amino-3-(4'-nitrophényle) propanoïque racémique

40,6 g d'acide 3-amino-3-(4'-nitrophényle) propanoïque racémique sont dissout dans 200 ml d'eau distillée et 55 ml de triéthylamine. 29,4 g d'anhydride glutarique sont ajoutés par petites portions et le mélange réactionnel est agité pendant 1 heure. Puis, le mélange réactionnel est acidifié avec 8,2 ml d'acide sulfurique à 95 % (poids/volume). Le précipité ainsi obtenu est filtré, lavé 3 fois avec 15 ml d'eau distillée et séché jusqu'à poids constant à 55°C sous vide. On obtient ainsi 52,84 g d'acide 3-(glutarylamide)-3-(4'-nitrophényle) propanoïque racémique.
La nature du produit obtenu a été déterminée par HPLC (Chromatographie Liquide Haute Performance).

b) Déacylation enzymatique par la glutaryl 7-ACA acylase cristallisée, en suspension (réacteur 100 ml)

20 g de l'acide obtenu à l'étape précédente sont dissout dans 75 ml d'eau distillée. Le pH de la suspension est ajusté à 8,2 en ajoutant 11,2 ml de soude à 30 % (poids/volume). On ajoute à cette solution 826 mg (626 unités) d'une suspension de glutaryl 7-ACA acylase cristallisée. Le mélange réactionnel est agité pendant 51 heures à 35°C et à pH contrôlé et ajusté entre 7,9 et 8,1. A la fin de la réaction, le mélange réactionnel est refroidi à 20°C et le pH est ajusté à 7,0 par ajout d'acide chlorhydrique 5N. On ajoute alors 20 ml d'éthanol. Le précipité d'acide (R) 3-amino-3-(4'-nitrophényle) propanoïque ainsi obtenu est filtré, lavé 3 fois avec 30 ml d'éthanol et séché jusqu'à poids constant à 45°C

sous vide. On obtient ainsi 5,65 g d'acide (R) 3-amino-3-(4'-nitrophényle) propanoïque avec un excès énantiomérique supérieur à 99 %.

La liqueur mère est acidifiée avec 17,5 ml d'acide chlorhydrique 5N. Le précipité qui se forme est filtré et lavé 2 fois avec 10 ml d'eau distillée. Le gâteau obtenu est séché jusqu'à poids constant à 45°C sous vide. On obtient ainsi 8,25 g d'acide (S) 3-(glutarylamide)-3-(4'-nitrophényle) propanoïque et d'acide (R) 3-(glutarylamide)-3-(4'-nitrophényle) propanoïque dans un rapport 91:9. La nature des produits obtenus a été déterminée par HPLC.

c) Déacylation enzymatique par la glutaryl 7-ACA acylase immobilisée obtenue de Roche Diagnostic GmbH (réacteur 100 ml)

20,5 g de l'acide obtenu à l'étape a) sont dissout dans 75 ml d'eau distillée. Le pH de la suspension est ajusté à 8,2 en ajoutant 11,9 ml d'hydroxyde de sodium à 30 % (poids/volume). On ajoute à cette solution 6,2 g (632,4 unités) de glutaryl 7-ACA acylase immobilisée humide (Roche Diagnostic GmbH). Le mélange réactionnel est agité pendant 18 heures à 35°C et à pH contrôlé et ajusté entre 7,9 et 8,1. A la fin de la réaction, le pH du mélange réactionnel est ajusté à 9,5 par ajout d'hydroxyde de sodium à 30 % (poids/volume) et le volume du mélange réactionnel est ajusté à 200 ml de façon à dissoudre l'acide (R) 3-amino-3-(4'-nitrophényle) propanoïque produit. L'enzyme immobilisée est enlevée par filtration et le pH de la liqueur mère est ajusté à 7,0 par ajout d'acide chlorhydrique 5N. On ajoute alors 50 ml d'éthanol au mélange réactionnel. L'acide (R) 3-amino-3-(4'-nitrophényle) propanoïque précipité est filtré, lavé avec 10 ml d'éthanol et séché jusqu'à poids constant à 45°C sous vide. On obtient ainsi 5,375 g d'acide (R) 3-amino-3-(4'-nitrophényle) propanoïque avec un excès énantiomérique égal à 98 %.

La liqueur mère est acidifiée avec 20 ml d'acide chlorhydrique 5N. Le précipité qui se forme est filtré et lavé 2 fois avec 15 ml d'eau distillée. Le gâteau est séché jusqu'à poids constant à 45°C sous vide. On obtient ainsi 6 g d'acide (S) 3-(glutarylamide)-3-(4'-nitrophényle) propanoïque dans un rapport 94:6.

La nature des produits obtenus a été déterminée par HPLC.

d) Déacylation enzymatique par la glutaryl 7-ACA acylase cristallisée en suspension (réacteur 500 ml)

100 g d'acide 3-(glutarylamide)-3-(4'-nitrophényle) propanoïque racémique obtenus comme dans l'étape a) sont dissout dans 400 ml d'eau distillée. Le pH de la suspension est ajusté à 8,2 en ajoutant 60 ml de soude à 30 % (poids/volume). On ajoute à cette solution 6,4 g (4653 unités) d'une suspension de glutaryl 7-ACA acylase cristallisée. Le mélange réactionnel est agité pendant 30 heures à 35°C et à pH contrôlé et ajusté entre 7,9 et 8,1. A la fin de la réaction, le mélange réactionnel est refroidi à 20°C et le pH est ajusté à 13 par ajout de 25 ml de soude à 30 % (poids/volume) de façon à dissoudre l'acide (R) 3-amino-3-(4'-nitrophényle) propanoïque formé. Les particules insolubles sont filtrées et le pH de la liqueur mère est ajusté à 7,0 par ajout de 27 ml d'acide chlorhydrique à 36 %. On ajoute alors 100 ml d'éthanol au mélange réactionnel. Le précipité d'acide (R) 3-amino-3-(4'-nitrophényle) propanoïque obtenu est filtré, lavé 2 fois avec 100 ml d'éthanol et séché jusqu'à poids constant à 45°C sous vide. On obtient ainsi 28,87 g d'acide (R) 3-amino-3-(4'-nitrophényle) propanoïque avec un excès énantiomérique égal à 97 %.

La nature du produit obtenu a été déterminée par HPLC.

**Exemple 2 : Résolution de l'acide 3-amino-3-phényle pronanoïque sous forme de mélange racémique**

a) Acylation de l'acide 3-amino-3-phényle propanoïque racémique

488 g d'acide 3-amino-3-phényle propanoïque racémique sont dissout dans 2 litres d'eau distillée contenant 236 g de soude sous forme de pastilles. On ajoute par petites portions en 1 heure 437,5 g d'anhydride glutarique au mélange réactionnel, sous agitation et à 20°C. Après 1 heure, le mélange réactionnel est acidifié avec 236 ml d'acide sulfurique à 95 % (poids/volume) et refroidi à 10°C. Le précipité ainsi formé est filtré puis lavé 3 fois avec 600 ml d'eau distillée. Le gâteau humide de 1610 g ainsi obtenu contient 602 g d'acide 3-glutarylamide-3-phényle propanoïque racémique. La nature des produits obtenus a été déterminée par HPLC.

b) Déacylation enzymatique par la glutaryl 7-ACA acylase cristallisée en suspension (réacteur 5 litres)

1575 g du gâteau humide obtenu précédemment contenant 589 g d'acide 3-glutarylamide-3-phényle propanoïque racémique sont dissout dans 1610 ml d'eau distillée. Le pH de la suspension est ajusté à 8,0 en ajoutant 440 ml de soude à 30 % (poids/volume). On ajoute à cette solution 52,6 g (32000 unités) d'une suspension de glutaryl 7-ACA acylase cristallisée. Le mélange réactionnel est agité pendant 41 heures à 35°C et à pH contrôlé et ajusté entre 7,9 et 8,1. A la fin de la réaction, le mélange réactionnel est refroidi à 20°C et le pH est ajusté à 13 par ajout de 25 ml d'hydroxyde de sodium à 30 % (poids/volume) de façon à dissoudre l'acide (R) 3-amino-3-(4'-nitrophényle) propanoïque

produit. Les particules insolubles sont filtrées et le pH de la liqueur mère est ajusté à 7,0 par ajout de 27 ml d'acide chlorhydrique à 36%.

Le précipité d'acide (R) 3-amino-3-phényle propanoïque ainsi obtenu est filtré, lavé avec 150 ml d'eau distillée et séché jusqu'à poids constant à 45°C sous vide. On obtient ainsi 96,9 g d'acide (R) 3-amino-3-phényle propanoïque avec un excès énantiomérique égal à 98 %.

La liqueur mère est acidifiée avec 180 ml d'acide sulfurique à 95 % (poids/volume). Le précipité ainsi formé est filtré et lavé 2 fois avec 400 ml d'eau distillée refroidie. Le gâteau est séché jusqu'à poids constant à 45°C sous vide. On obtient ainsi 314,6 g d'acide (S) 3-(glutarylamide)-3-phényle propanoïque et d'acide (R) 3-(glutarylamide)-3-phényle propanoïque dans un rapport 90:10.

La nature des produits obtenus a été déterminée par HPLC.

c) Déacylation de l'acide (S) 3-glutarylamide-3-phényl propanoïque par hydrolyse basique

557,2 g d'un mélange d'acide (S) 3-glutarylamide-3-phényl propanoïque et d'acide (R) 3-glutarylamide-3-phényl propanoïque dans un rapport 90:10 obtenus à l'étape b) précédente sont dissout dans 3;91 litres d'eau distillée et 1,65 litres de soude à 30 % (poids/volume). Le mélange réactionnel est agité pendant 4 jours à 70°C.

Puis, le mélange réactionnel est refroidi à 15°C. Le produit obtenu est précipité en ajustant le pH à 6,9 par ajout de 3,21 litres d'acide chlorhydrique à 37 % (poids/volume), filtré et séché jusqu'à poids constant à 45°C sous vide. On obtient ainsi 202,4 g d'acide (S) 3-amino-3-phényl propanoïque et d'acide (R) 3-amino-3-phényl propanoïque avec un excès énantiomérique supérieur à 98 %.

La nature des produits obtenus a été déterminée par HPLC.

## Revendications

1. Procédé de séparation des énantiomères d'un acide aminé ou ester d'acide aminé; qui consiste à traiter un mélange racémique dudit acide aminé avec de l'anhydride glutarique puis avec l'enzyme glutaryl 7-ACA acylase de sorte que l'on récupère l'un des énantiomères dudit acide aminé, l'autre énantiomère restant sous forme de dérivé de l'amide glutaryle correspondant

2. Procédé selon la revendication 1 **caractérisé en ce que** ledit acide amine ou ester d'acide aminé pour formule générale :

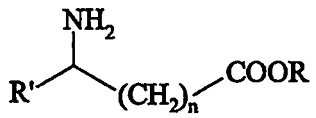

(I)

dans laquelle

- n est un entier choisi parmi 0, 1, 2, 3, 4, 5 ou 6,
- R représente un atome d'hydrogène ou bien un radical alkyle, alcène, alcyne, cycloalkyle, aryle, un hydrocarbure polycyclique condensé, ou un hétérocycle, tous ces radicaux étant éventuellement substitués,
- et R' représente un radical alkyle, alcène, alcyne, cycloalkyle, aryle, un hydrocarbure polycyclique condensé, un hétérocycle, ou bien un radical oxy, thio, sulfoxyde ou sulfonyle substitué par un groupe alkyle, aryle, cycloalkyle ou un hétérocyle, tous ces radicaux étant en outre éventuellement substitués.

3. Procédé selon la revendication 2 **caractérisé en ce que** ledit acide aminé ou ester d'acide aminé est choisi parmi les composés de formule générale (I) :

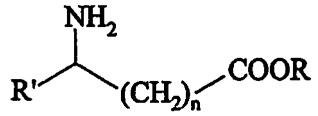

(I)

dans laquelle n est un entier choisi parmi 0, 1, 2 ou 3, R représente un atome d'hydrogène ou bien un radical alkyle ou aryle et R' est tel que défini dans la revendication 2.

**4.** Procédé selon la revendication 2 ou 3 **caractérisé en ce que** ledit acide aminé ou ester d'acide aminé est choisi parmi les composés de formule générale (I) :

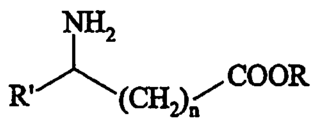

(I)

dans laquelle n est un entier égal à 0, 1 ou 2, R représente un atome d'hydrogène ou bien une fonction alkyle et R' est choisi parmi les aryles ou les hétérocycles éventuellement substitués

**5.** Procédé selon l'une des revendications précédentes **caractérisé en ce que** l'enzyme glutaryl 7-ACA acylase est utilisée sous forme soluble ou immobilisée.

**6.** Procédé selon l'une des revendications précédentes **caractérisé en ce que** la quantité d'enzymes mise en oeuvre par rapport à la quantité totale d'acide aminé ou ester d'acide aminé de départ (substrat) est comprise 1 et 100 unités par mmole de substrat.

**7.** Procédé selon l'une des revendications précédentes **caractérisé en ce que** la réaction est effectuée en milieu aqueux tamponné.

**8.** Procédé selon la revendication 7 **caractérisé en ce que** ledit tampon aqueux a une concentration comprise entre 10 mM et 200 mM et est choisi parmi les tampons acétate utilisables à pH compris entre 5 et 6,5 ou les tampons phosphate utilisables à pH compris entre 6,5 et 8, ou les tampons pyrophosphate utilisables à pH compris entre 8 et 9.

**9.** Procédé selon l'une des revendications précédentes **caractérisé en ce que** le pH est contrôlé et ajusté entre 6 et 9.

**10.** Procédé selon l'une des revendications précédentes **caractérisé en ce que** le traitement de l'acide aminé ou ester d'acide aminé, par l'anhydride glutarique est mis en oeuvre à une température comprise entre 20°C et 40°C.

**11.** Procédé selon l'une des revendications précédentes **caractérisé en ce que** le traitement par l'enzyme glutaryl 7-ACA acylase est effectuée à une température comprise entre 10°C et 50°C.

**12.** Procédé selon l'une des revendications précédentes **caractérisé en ce que** le temps de réaction varie entre 1 heure et 100 heures.

**13.** Procédé selon l'une des revendications précédentes **caractérisé en ce que** l'on sépare en outre les énantiomères (R) et (S).

**14.** Procédé selon la revendication 13 **caractérisé en ce que** ladite séparation des énantiomères (R) et (S) est effectuée par filtration, extraction, chromatographie ou cristallisation.

**15.** Procédé selon l'une des revendications 13 ou 14 **caractérisé en ce que** l'énantiomère dérivé de l'amide glutaryle qui a été séparé est en outre hydrolysé de sorte que l'on récupère l'acide aminé correspondant sous forme d'énantiomère.

## Claims

**1.** Process for separating the enantiomers of an amino acid or amino acid ester, which consists in treating a racemic mixture of the said amino acid with glutaric anhydride and then with the enzyme glutaryl-7-ACA acylase so as to recover one of the enantiomers of said amino acid, the other enantiomer remaining in the form of the corresponding glutarylamide derivative.

2. Process according to Claim 1 **characterized in that** said amino acid or amino acid ester has the general formula:

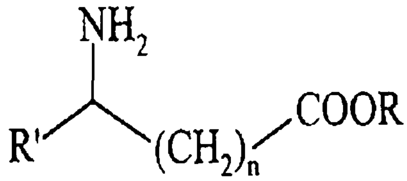

(I)

in which

- n is an integer selected from 0, 1, 2, 3, 4, 5 or 6,
- R is a hydrogen atom or else an alkyl, alkene, alkyne, cycloalkyl, aryl radical, a condensed polycyclic hydrocarbon, or a heterocycle, all of these radicals being optionally substituted,
- and R' is an alkyl, alkene, alkyne, cycloalkyl, aryl radical, a condensed polycyclic hydrocarbon, a heterocycle, or else an oxy, thio, sulphoxide or sulphonyl radical substituted by an alkyl, aryl, cycloalkyl group or a heterocycle, all of these radicals, moreover, being optionally substituted.

3. Process according to Claim 2 **characterized in that** said amino acid or amino acid ester is selected from the compounds of general formula (I):

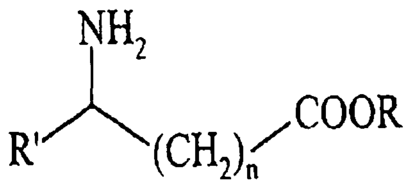

(I)

in which n is an integer selected from 0, 1, 2, or 3, R is a hydrogen atom or else an alkyl or aryl radical and R' is as defined in Claim 2.

4. Process according to Claim 2 or 3 **characterized in that** said amino acid or amino acid ester is selected from the compounds of general formula (I):

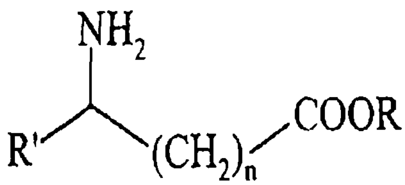

(I)

in which n is an integer equal to 0, 1 or 2, R is a hydrogen atom or else an alkyl function and R' is selected from optionally substituted heterocycles or aryls.

5. Process according to one of the preceding claims **characterized in that** the enzyme glutaryl-7-ACA acylase is used in soluble or immobilized form.

6. Process according to any of the previous claims **characterized in that** the amount of enzyme used relative to the total amount of initial amino acid or amino acid ester (substrate) is between 1 and 100 units per mmole of substrate.

7. Process according to any of the previous claims **characterized in that** the reaction is carried out in a buffered aqueous medium.

8. Process according to Claim 7 **characterized in that** said aqueous buffer has a concentration of between 10 mM and 200 mM and is selected from acetate buffers which can be used at a pH of between 5 and 6.5 or phosphate buffers which can be used at a pH of between 6.5 and 8, or pyrophosphate buffers which can be used at a pH of between 8 and 9.

9. Process according to any of the previous claims **characterized in that** the pH is monitored and adjusted to between 6 and 9.

10. Process according to any of the previous claims **characterized in that** the treatment of the amino acid or amino acid ester with glutaric anhydride is implemented at a temperature of between 20°C and 40°C.

11. Process according to any of the previous claims **characterized in that** the treatment with the enzyme glutaryl-7-ACA acylase is carried out at a temperature of between 10°C and 50°C.

12. Process according to any of the previous claims **characterized in that** the reaction time is comprised between 1 hour and 100 hours.

13. Process according to any of the previous claims **characterized in that** additionally the (R) and (S) enantiomers are separated.

14. Process according to Claim 13 **characterized in that** said separation of the (R) and (S) enantiomers is carried out by filtration, extraction, chromatography or crystallization.

15. Process according to one of Claims 13 or 14 **characterized in that** the enantiomer derived from the glutarylamide which has been separated is additionally hydrolysed so as to recover the corresponding amino acid in enantiomeric form.

**Patentansprüche**

1. Verfahren zur Trennung von Enantiomeren einer Aminosäure oder eines Aminosäureesters, das darin besteht, daß man eine razemische Mischung dieser Aminosäure mit Glutarsäureanhydrid und anschließend mit Enzym Glutaryl-7-ACA-acylase so behandelt, daß man das eine Enantiomer dieser Aminosäure gewinnt, während das andere Enantiomer in Form eines Derivats des entsprechenden Glutarylamids zurückbleibt.

2. Verfahren nach Anspruch 1, **dadurch gekennzeichnet, daß** die Aminosäure oder der Aminosäureester der allgemeinen Formel

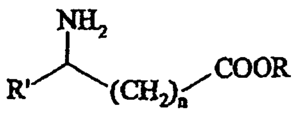

(I)

entspricht, in der

- n eine ganze Zahl aus der Reihe 0, 1, 2, 3, 4, 5 oder 6 bedeutet,
- R ein Wasserstoffatom oder einen Alkyl-, Alken-, Alkin-, Cycloalkyl-, Arylrest, einen kondensierten polycyclischen Kohlenwasserstoff oder einen Heterocyclus bedeutet, wobei alle diese Reste gegebenenfalls substituiert sind,
- und R' einen Alkyl-, Alken-, Alkin-, Cycloalkyl-, Arylrest, einen kondensierten polycyclischen Kohlenwasserstoff, einen Heterocyclus oder einen durch eine Alkyl-, Aryl-, Cycloalkylgruppe oder einen Heterocyclus substituierten Oxy-, Thio-, Sulfoxid- oder Sulfonylrest bedeutet, wobei alle diese Reste gegebenenfalls weiter substituiert sind.

3. Verfahren nach Anspruch 1, **dadurch gekennzeichnet, daß** die Aminosäure oder der Aminosäureester der allgemeinen Formel (I)

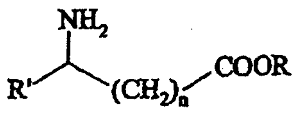 (I)

entspricht, in der n eine ganze Zahl aus der Reihe 0, 1, 2 oder 3 bedeutet, R ein Wasserstoffatom oder einen Alkyl- oder Arylrest bedeutet und R' wie in Anspruch 2 definiert ist.

4. Verfahren nach Anspruch 1, **dadurch gekennzeichnet, daß** die Aminosäure oder der Aminosäureester der allgemeinen Formel (I)

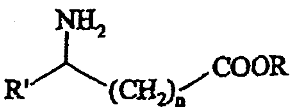 (I)

entspricht, in der n eine ganze Zahl aus der Reihe 0, 1 oder 2 bedeutet, R ein Wasserstoffatom oder eine Alkylfunktion bedeutet und R' aus der Reihe der gegebenenfalls substituierten Aryle oder Heterocyclen stammt.

5. Verfahren nach einem der vorhergehenden Ansprüche, **dadurch gekennzeichnet, daß** das Enzym Glutaryl-7-ACA-acylase in löslicher oder immobilisierter Form verwendet wird.

6. Verfahren nach einem der vorhergehenden Ansprüche, **dadurch gekennzeichnet, daß** die verwendete Enzymmenge im Vergleich zu der Gesamtausgangsmenge der Aminosäure oder des Aminosäureesters (Substrat) zwischen 1 und 100 Einheiten pro mmol Substrat beträgt.

7. Verfahren nach einem der vorhergehenden Ansprüche, **dadurch gekennzeichnet, daß** die Reaktion in einem gepufferten wäßrigen Medium durchgeführt wird.

8. Verfahren nach Anspruch 7, **dadurch gekennzeichnet, daß** der wäßrige Puffer eine Konzentration zwischen 10 mM und 200 mM aufweist und aus der Reihe der Acetatpuffer, die zwischen pH 5 und pH 6,5 verwendet werden können, oder der Phosphatpuffer, die zwischen pH 6,5 und pH 8 verwendet werden können, oder der Pyrophosphatpuffer, die zwischen pH 8 und pH 9 verwendet werden können, stammt.

9. Verfahren nach einem der vorhergehenden Ansprüche, **dadurch gekennzeichnet, daß** der pH-Wert kontrolliert und zwischen 6 und 9 eingestellt wird.

10. Verfahren nach einem der vorhergehenden Ansprüche, **dadurch gekennzeichnet, daß** die Behandlung der Aminosäure oder des Aminosäureesters mit dem Glutarsäureanhydrid bei einer Temperatur zwischen 20°C und 40°C durchgeführt wird.

11. Verfahren nach einem der vorhergehenden Ansprüche, **dadurch gekennzeichnet, daß** die Behandlung mit dem Enzym Glutaryl-7-ACA-acylase bei einer Temperatur zwischen 10°C und 50°C durchgeführt wird.

12. Verfahren nach einem der vorhergehenden Ansprüche, **dadurch gekennzeichnet, daß** die Reaktionszeit zwischen 1 Stunde und 100 Stunden schwankt.

13. Verfahren nach einem der vorhergehenden Ansprüche, **dadurch gekennzeichnet, daß** man weiterhin die (R)- und die (S)- Enantiomere trennt.

14. Verfahren nach Anspruch 13, **dadurch gekennzeichnet, daß** die Trennung der (R)- und der (S)- Enantiomere mittels Filtration, Extraktion, Chromatographie oder Kristallisation durchgeführt wird.

15. Verfahren nach Anspruch 13 oder 14, **dadurch gekennzeichnet, daß** das abgetrennte, von Glutarylamid abgeleitete Enantiomer weiterhin so hydrolysiert wird, daß man die entsprechende Aminosäure in Form ihres Enantiomers erhält.

## FIG. 1

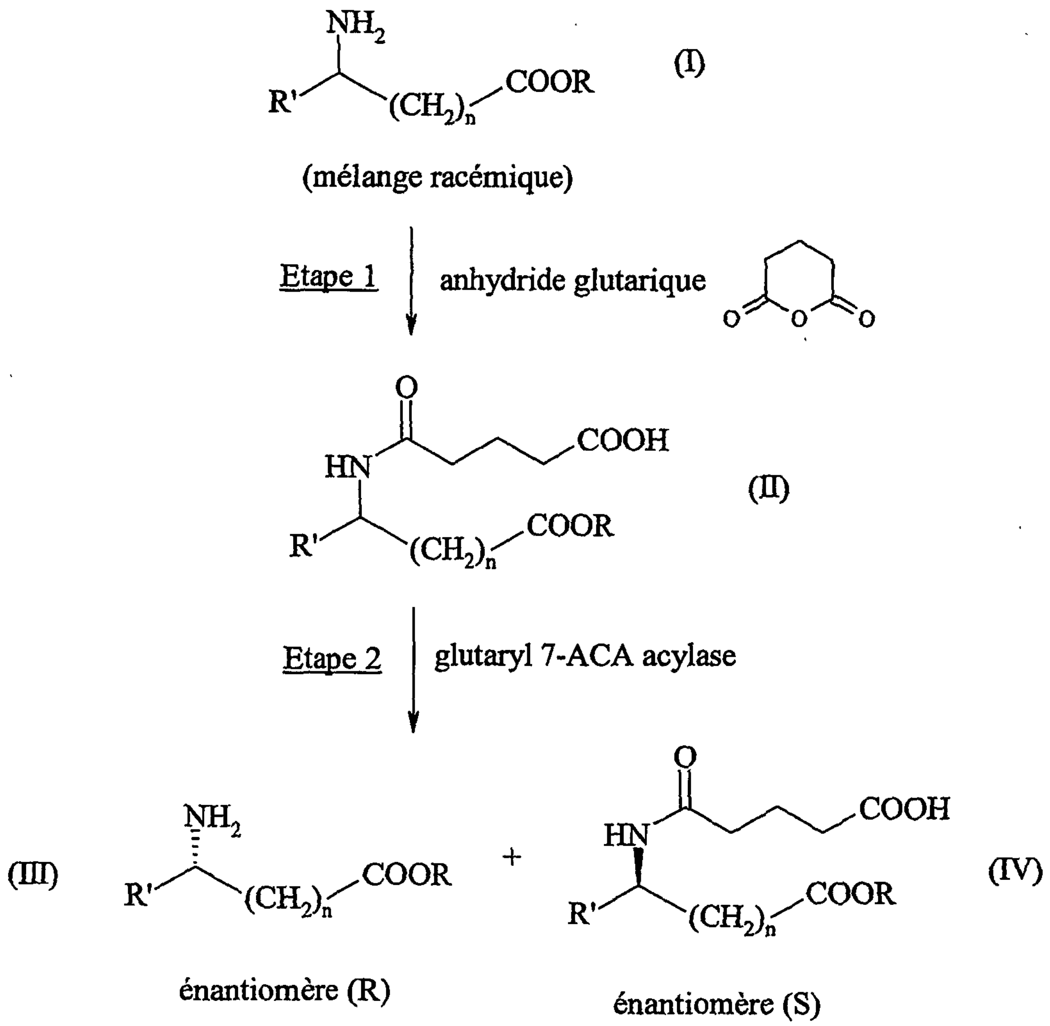


**ou bien selon la nature du radical R':**

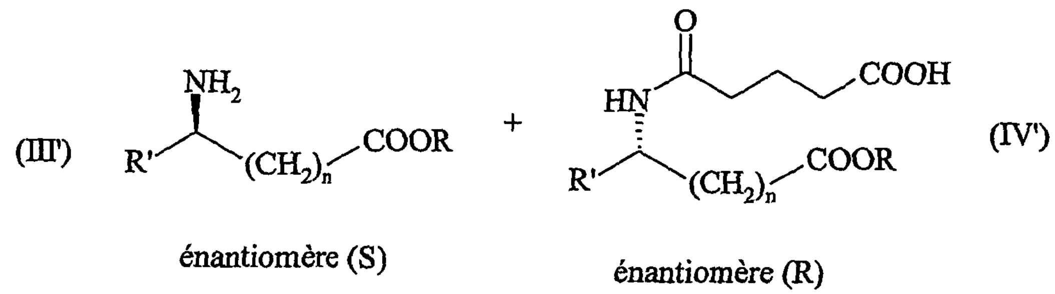

**FIG. 2**

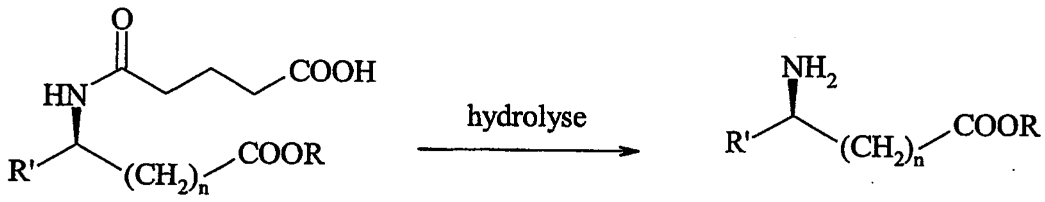


énantiomère (S) énantiomère (S)

(IV) (V)

**ou bien :**

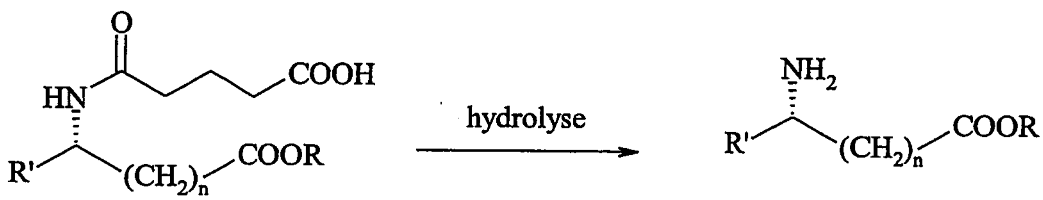


énantiomère (R) énantiomère (R)

(IV') (V')